# EUROPEAN PATENT APPLICATION

(11) **EP 2 407 469 A1**
(43) Date of publication of application: **18.01.2012**
(21) Application number: 10169397.6
(22) Date of filing: 13.07.2010
(51) Int. Cl.: C07D 487/04, C07D 239/557, A61K 31/4985, A61P 3/10

(54) **Salt of sitagliptin**

(71) Applicant: Chemo Ibérica, S.A., 08028 Barcelona (ES)
(72) Inventor: Ventimiglia, Gianpiero, 72021 Francavilla Fontana (IT); Magrone, Domenico, 20128 Milano (IT); Bravin, Fabio Massimo, 20126 Milano (IT)
(74) Representative: Palladino, Saverio Massimo

(57) **Abstract**

The present invention is directed to a novel salt of sitagliptin with orotic acid, a solid form of this novel salt, and processes for the preparation thereof.

## Description

### Field of the invention

The present invention relates to a novel salt of sitagliptin, a solid form of this novel salt, and processes for preparation thereof.

### Background of the invention

Sitagliptin, 7-[(3*R*)-3-amino-1-oxo-4-(2,4,5-trifluorophenyl)butyl]-5,6,7,8-tetrahydro-3-(trifluoromethyl)-1,2,4-triazolo[4,3-a]pyrazine, is a compound of formula (I):

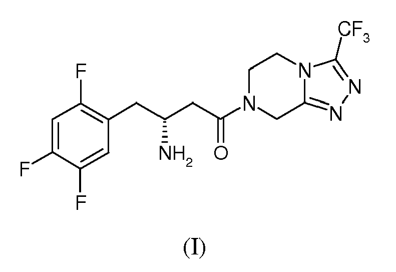

Sitagliptin, disclosed in US Pat. No. 6,699,871 B2 and in international application WO 03/004498 A1, belongs to a class of beta-amino tetrahydrotriazolo-[4,3-a]pyrazines, which are potent second generation inhibitors of dipeptidyl-peptidase IV (DP-IV), and therefore useful for the treatment of Type-2 diabetes. The preparation of the compound is also described in the paper "Highly Efficient Asymmetric Synthesis of Sitagliptin", Hansen et al.: Journal of the American Chemical Society (2009), 131(25), 8798-8804; once obtained, the compound can be purified according to methods standard in the field, obtaining sitagliptin of purity suitable for pharmaceutical applications.

Sitagliptin is marketed as monophosphate salt and monohydrate adduct under the trade name Januvia^{®} (Trademark of Merck & Co.).

Several salts of sitagliptin are disclosed in the patent literature. Although pharmaceutically acceptable salts are encompassed within the scope of US 6,699,871 B2 and WO 03/004498 A1, international patent application WO 2005/072530 A1 concerns with novel crystalline salts of sitagliptin with hydrochloric acid, benzenesulfonic acid, p-toluenesulfonic acid, tartaric acid and camphorsulfonic acid.

WO 2009/085990 A2 discloses salts of sitagliptin with sulphuric acid, hydrobromic acid, methanesulfonic acid, acetic acid, benzoic acid, oxalic acid, succinic acid, mandelic acid, fumaric acid and lactic acid, obtained by means of chemical reaction between sitagliptin free base and the corresponding acid in 2-propanol.

WO 2007/035198 A2 discloses sitagliptin in the form of salt with dodecylsulfonic acid, obtained by ionic exchange with sodium dodecylsulphate in water.

Besides the chemical differences, the different salts of a substance possess different energies of the crystal lattice and, thus, they show different physical properties of the solid state such as form, density, melting point, colour, stability, dissolution rate, milling facility, granulation, compacting etc. These differences in morphology may have relevant effects on the flowability of the milled solid (flowability affects the ease with which the material is handled during processing into a pharmaceutical product; when particles of the powdered compound do not flow past each other easily, a formulation specialist must necessitate the use of glidants), development, transport and storage stability of individual administration forms, on the ability to produce different administration forms, on their application, on the solubility in polar or non-polar, protic or aprotic solvents, on solubility in aqueous solution, on solubility in the gastric juices, on solubility in blood serum, and finally on bio-availability. The rate of dissolution of an active ingredient in a patient's stomach fluid can have therapeutic consequences since it imposes an upper limit on the rate at which an orally-administered active ingredient can reach the patient's bloodstream. The rate of dissolution is also a consideration in formulating syrups, elixirs and other liquid medicaments.

From a physical point of view, once salts of pharmaceutical compounds have been isolated, they can be characterized by their thermal behaviour. Thermal behavior is measured by such techniques as Differential Scanning Calorimetry (DSC), and can be used to distinguish a pharmaceutically acceptable salt from others. Melting points, glass transitions, crystallinity, solvates, and/or presence of polymorphs may be evidenced by multiple endotherms, poorly defined endotherms, and endotherms near the boiling point of solvents. Additionally, a melting or glass transition point higher than 125 °C is preferred to avoid incompatibility with the mechanical forces typically encountered during the manufacture of drug product such as compaction or heat of mixing as a function of shear force.

The potential for polymorphism may also give rise to distinct spectroscopic properties that may be detectable by X-Ray Powder Diffraction (XRPD) analysis.

The discovery of novel pharmaceutically acceptable salts of active pharmaceutical ingredients (APIs) provides a new opportunity to improve the performance characteristics of a pharmaceutical product. It enlarges the repertoire of materials that a formulation scientist has available for designing a pharmaceutical dosage form, or a drug with a targeted release profile, or other desired characteristics, such as flowability and suitable rate of dissolution in aqueous fluid.

Criteria for acid selection in order to obtain a new salt of a pharmaceutical compound comprise employment of acids that are generally regarded as safe (GRAS) and have previously been used in other marketed drugs. Additionally, the acids would need to be of sufficient strength to protonate the corresponding free base of the title compound. Further, preparation and characterization of novel salts of APIs can provide new commercial opportunities for a pharmaceutical company. Finally, use of eco-compatible chemicals and processes, suitable for large scale preparations, is desirable.

On the basis of these considerations, preparation and characterization of a novel salt of sitagliptin is desirable.

An object of the present invention is to provide a novel salt of sitagliptin with orotic acid, herein below referred to as sitagliptin orotate, sitagliptin orotate in amorphous form, and processes for preparing them.

### Summary of the invention

The present invention provides a novel salt of sitagliptin with orotic acid, herein below referred to as sitagliptin orotate, sitagliptin orotate hydrates and polymorphs, sitagliptin orotate in amorphous form, and processes for preparing them.

In one embodiment, the invention provides a novel salt of sitagliptin with orotic acid, herein below referred to as sitagliptin orotate.

In another embodiment, the invention provides a process for preparing sitagliptin orotate comprising the steps of:
a) suspending sitagliptin free base in water;
b) adding orotic acid in order to obtain a clear solution;
c) heating the solution in order to promote the reaction of orotic acid with sitagliptin free base;
d) cooling down or allowing to cool the solution to a temperature comprised in a range of about 15 °C to about 25 °C;
e) extracting residual sitagliptin free base by means of an organic solvent immiscible with water;
f) separating the aqueous phase and filtering it through a celite pad;
g) lyophilizing the aqueous solution.

In another embodiment, the invention provides sitagliptin orotate in amorphous form.

### Brief description of the drawings and instrumental skills

Figure 1 provides DSC thermogram of amorphous sitagliptin orotate;
Figure 2 provides XRPD pattern of amorphous sitagliptin orotate.

### Detailed description of the invention

All terms as used in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition. The term "about" encompasses the range of experimental error that may typically occur in a measurement.

The present inventors have now found that sitagliptin may be isolated as a novel salt derivative with orotic acid which is until now unknown. Orotic acid has the IUPAC name 1,2,3,6-tetrahydro-2,6-dioxo-4-pyrimidinecarboxylic acid and formula (II):

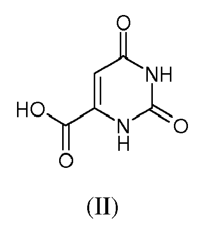

In the conditions of the process of the invention, orotic acid protonates the amino group of sitagliptin, giving rise to a salt formed by ionic attraction between the sitagliptin-derived cation and the orotate anion. The resulting salt can be represented by formula (III) below, in which the dotted line indicates the zone of attraction between the two ions:

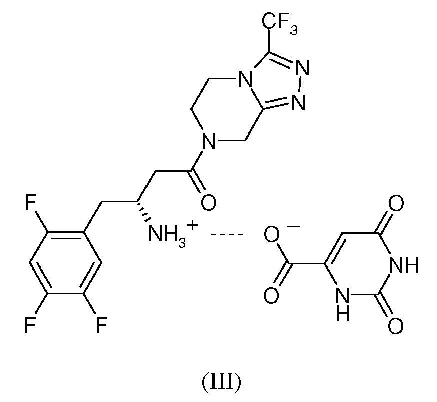

The novel salt of sitagliptin is found to be stable at room temperature, reproducible and suitable for pharmaceutical preparations. It can be prepared with efficient and economic process particularly suited to large-scale preparation.

In another embodiment, the invention provides a process for the preparation of sitagliptin orotate comprising the steps of:
a) suspending sitagliptin free base in water at a suitable temperature. Preferably, the temperature is comprised in a range of about 10 °C to about 30 °C; more preferably, the temperature is comprised in a range of about 15 °C to about 25 °C. Preferably, sitagliptin free base is in a ratio of about 1:10 to about 1:15 weight/volume with respect to water;
b) adding orotic acid in order to obtain a clear solution. Preferably, orotic acid is used as anhydrous and/or monohydrate adduct; more preferably, orotic acid is used as monohydrate adduct; preferably, orotic acid is in a ratio of about 0.9:1 to about 0.95:1 mole/mole with respect to sitagliptin;
c) heating the solution obtained in step b) up to a temperature preferably comprised in a range of about 30 °C to about 40 °C in order to promote the reaction between orotic acid and sitagliptin free base;
d) cooling down the solution with suitable means, or allowing the solution to freely cool down, to a temperature comprised in a range of about 15 °C to about 25 °C;
e) extracting residual sitagliptin free base by means of an organic solvent immiscible with water. Preferably, the organic solvent immiscible with water is selected from the group consisting of toluene, dichloromethane, ethyl acetate, isopropyl acetate, butyl acetate, diisopropylether, methyl *tert*-butyl ether (MTBE), 1-butanol, methyl ethyl ketone, methyl isobutyl ketone. Preferably, the organic solvent is in a ratio of about 1:4 to about 1:6 volume/volume with respect to water;
f) separating the aqueous phase by means of methods well known to those skilled in the art;
g) lyophilizing the aqueous solution thus obtained by means of methods well known to those skilled in the art, at temperature preferably comprised in a range of about 15 °C to about 25 °C.

In another embodiment, the invention provides sitagliptin orotate in amorphous form, characterized by means of XRPD analysis and DSC thermal analysis.

XRPD pattern shows the typical profile of an amorphous solid. The DSC thermogram evidences a broad endotherm with a maximum peak at about 83 °C due to the loss of water, and a thermal phenomenon at about 144 °C, due to a glass transition or a similar phenomenon.

Sitagliptin orotate shows a residual water content comprised in a range of about 3.5% to about 5.5% by weight, as determined by means of Karl Fischer titration.

In the following, the present invention shall be illustrated by means of one example, which is not construed to be viewed as limiting the scope of the invention.

### Example 1

Sitagliptin free base, having HPLC purity of 99.9% and with an enantiomeric excess of the 3*R-*form higher than 99.9%, is prepared according to the Hansen *et al.* paper cited before. 1.6 g (3.9 mmol) of the compound are suspended in water (20 ml) at 20 °C and orotic acid monohydrate (0.64 g, 3.7 mmol of anhydrous orotic acid, Sigma-Aldrich) is added. The solution is warmed up to 35-37 °C, kept at this temperature for ten minutes, and then allowed to cool down spontaneously to 20-25 °C. The system is then extracted twice with dichloromethane (5 ml), and the resulting aqueous phase is separated, filtered through a celite pad and lyophilized at 25 °C, thus affording amorphous sitagliptine orotate (2.1 g), having a water content of 4.7% by weight as determined by Karl Fischer titration.

On the thus obtained product, XRPD analysis and DSC thermal analysis are carried out.

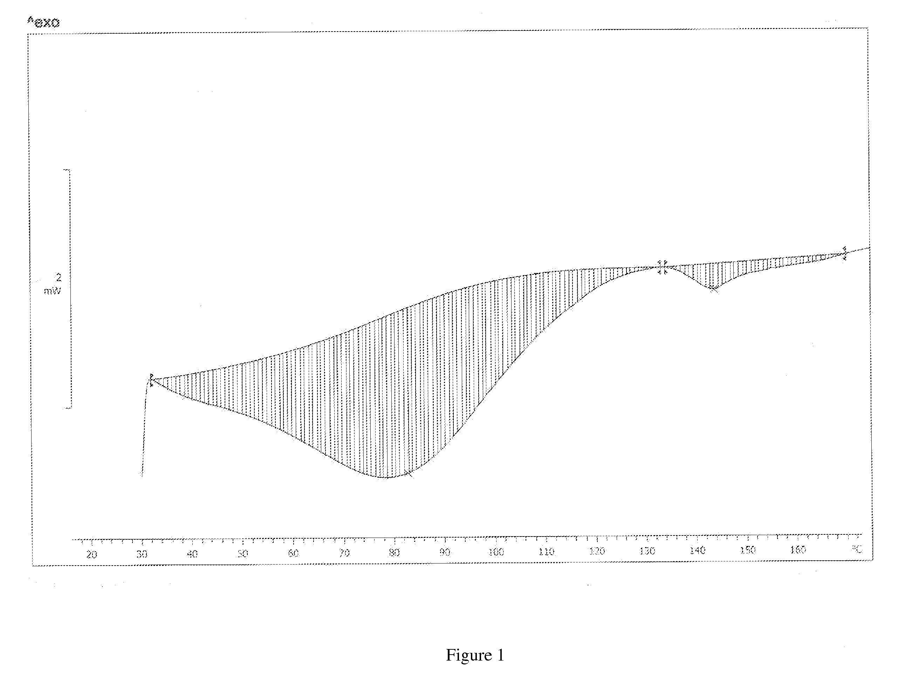

XRPD analysis is performed on a APD 2000 Ital Structures diffractometer at room temperature, using a CuKα tube (40 kV, 30 mA, λ = 1.5406 Å) as the X-ray source. Data collection is made in 2θ step scan mode, at a scan speed of 0.04°/s in the range of 3° to 40° in 2θ, using as sample approximately 100 mg of product accurately ground and placed on an aluminium sampler. The results of this test are reported in Figure 1, which shows the typical pattern of an amorphous material.

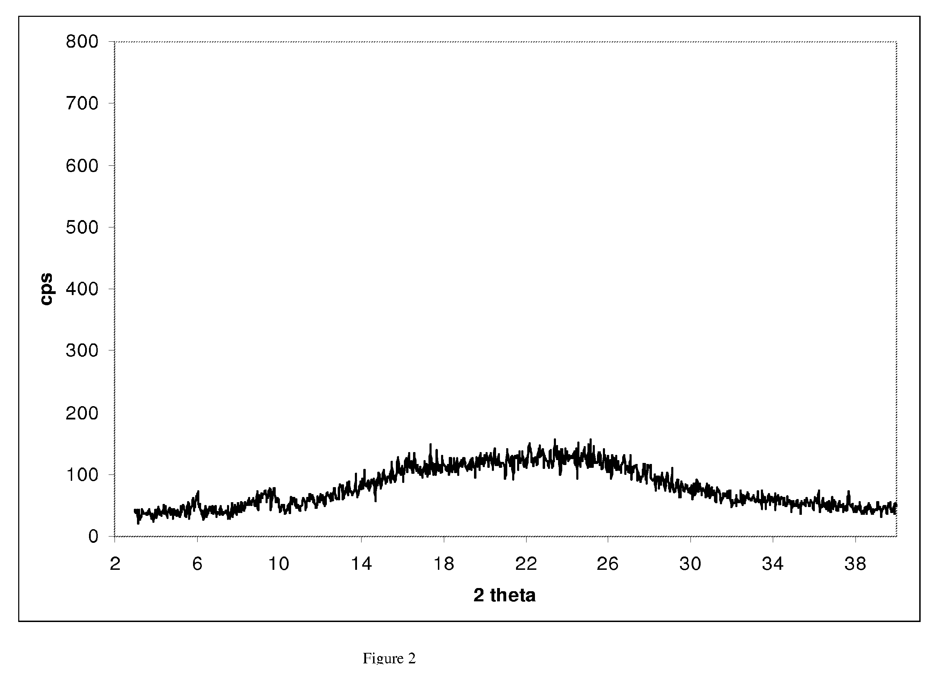

DSC thermal analysis is performed on a Mettler Toledo STAR^{e} 822 differential scanning calorimeter. Approximately 2-5 mg samples are placed in aluminium pans and heated from 30 to 170 °C in a dry nitrogen atmosphere at a heating rate of 10 °C/minute. The results of this test are reported in Figure 2. In the figure, the upper line of the graph is the "base line" of the test, namely, it represents the heat exchanged by an empty sample holder (equal to the one holding the sample under test), while the lower line represents the heat exchanged by the sample holder; the difference between these two lines represents the contribution to heat exchange of the sample, and the integral of the areas defined between the two lines is a measure of the heat exchanged by the sample. The DSC thermogram evidences a broad endotherm with a maximum peak at about 83 °C due to the loss of water; additionally, it's noteworthy to observe a thermal phenomenon at about 144 °C, due to a glass transition or a similar phenomenon.

## Claims

1. Sitagliptin orotate and hydrates and polymorphs thereof.

2. Sitagliptin orotate according to claim 1 in amorphous form.

3. Sitagliptin orotate according to claim 1, comprising between about 3.5% about 5.5% by weight of water.

4. Process for the production of sitagliptin orotate, comprising the steps of:
a) suspending sitagliptin free base in water;
b) adding orotic acid in order to obtain a clear solution;
c) heating the solution in order to promote the reaction of orotic acid with sitagliptin free base;
d) cooling down or allowing to cool the solution to a temperature comprised in a range of about 15 °C to about 25 °C;
e) extracting residual sitagliptin free base by means of an organic solvent immiscible with water;
f) separating the aqueous phase and filtering it through a celite pad;
g) lyophilizing the aqueous solution.

5. Process according to claim 4, in which in step a) the temperature is comprised in a range of about 10 °C to about 30 °C, preferably in a range of about 15 °C to about 25 °C.

6. Process according to claim 4, in which in step a) sitagliptin free base is added to water in a ratio of about 1:10 to about 1:15 weight/volume.

7. Process according to claim 4, in which in step b) orotic acid is used as anhydrous and/or monohydrate adduct.

8. Process according to claim 4, in which in step b) orotic acid is in a ratio of about 0.9 to about 0.95 mole per mole of sitagliptin.

9. Process according to claim 4, in which in step c) the solution obtained in step b) is heated up to a temperature comprised in a range of about 30 °C to about 40 °C.

10. Process according to claim 4, in which the organic solvent immiscible with water used in step e) is selected from the group consisting of toluene, dichloromethane, ethyl acetate, isopropyl acetate, butyl acetate, diisopropylether, methyl *tert*-butyl ether (MTBE), 1-butanol, methyl ethyl ketone, methyl isobutyl ketone.

11. Process according to claim 4, in which the organic solvent immiscible with water used in step e) is used in a ratio of about 1:4 to about 1:6 volume/volume with respect to water.

12. Process according to claim 4, in which the lyophilization of step g) is carried out at temperature comprised in a range of about 15 °C to about 25 °C.

13. Sitagliptin orotate according to any of claims 1 to 3 for use as a medicament.

14. Use of sitagliptin orotate according to any of claims 1 to 3 for the manufacture of a medicament for the treatment of Type-2 diabetes.

15. Pharmaceutical composition comprising sitagliptin orotate according to any of claims 1 to 3 in association with one or more pharmaceutically acceptable excipients or carriers.
